(19)

Europäisches
Patentamt
European
Patent Office
Office européen
des brevets

(11) **EP 3 239 284 A1**

(12) **EUROPEAN PATENT APPLICATION**
published in accordance with Art. 153(4) EPC

(43) Date of publication:
**01.11.2017 Bulletin 2017/44**

(21) Application number: **15872696.8**

(22) Date of filing: **07.12.2015**

(51) Int Cl.:
*C11D 3/20* (2006.01)     *A61K 8/19* (2006.01)
*A61K 8/34* (2006.01)     *A61K 8/46* (2006.01)
*C11D 1/29* (2006.01)     *C11D 3/04* (2006.01)

(86) International application number:
**PCT/JP2015/084311**

(87) International publication number:
**WO 2016/104127 (30.06.2016 Gazette 2016/26)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **26.12.2014 JP 2014266638**

(71) Applicant: **Kao Corporation
Tokyo 103-8210 (JP)**

(72) Inventor: **KAMENOUE, Shogo
Wakayama-shi
Wakayama 640-8580 (JP)**

(74) Representative: **Hoffmann Eitle
Patent- und Rechtsanwälte PartmbB
Arabellastraße 30
81925 München (DE)**

(54) **SURFACTANT COMPOSITION**

(57) The present invention provides a surfactant composition that is excellent in storage stability while maintaining high viscosity thereof even when the concentration of a surfactant used therein is low, and a process for producing the surfactant composition. The present invention relates to [1] a surfactant composition including (A): a polyol having from 2 to 3 carbon atoms; (B): a specific polyoxyalkylene alkyl or alkenyl ether sulfate or a salt thereof; (C): at least one compound selected from the group consisting of an alkali metal salt other than the aforementioned component (B) and the like; and (D): water, in which a content of the component (A) in the composition is from 25 to 98% by mass, a content of the component (B) in the composition is from 0.05 to 10% by mass in terms of the sulfate, a total content of alkali metals (X) derived from the polyoxyalkylene alkyl or alkenyl ether sulfate salt as the component (B) and derived from the at least one compound selected from the group consisting of the alkali metal salt and the like as the component (C), in the composition is not more than 2.5% by mass in terms of the metal elements, and a molar ratio of the alkali metals (X) to the component (B) is not less than 1.5, and [2] a process for producing the aforementioned composition.

**Description**

FIELD OF THE INVENTION

The present invention relates to a surfactant composition used for cosmetics, detergents, etc., and a process for producing the surfactant composition.

BACKGROUND OF THE INVENTION

As a method for increasing viscosity of an anionic surfactant aqueous solution, there is conventionally known the (thickening) method in which an inorganic salt such as sodium chloride and sodium sulfate is added to the aqueous solution to raise a concentration of the aqueous solution by agglomeration of micelles therein. This method has been generally used in extensive applications because of excellent dissolvability of the inorganic salt in the aqueous solution. However, the thickening effect of the inorganic salt on the aqueous solution is comparatively low in view of an amount of the inorganic salt added thereto. Therefore, in the aforementioned method, a large amount of the inorganic salt must be added to the aqueous solution for controlling the viscosity of the aqueous solution to a desired high value.

For example, JP 2009-120664A (Patent Literature 1) discloses a surfactant composition obtained by compounding compounds represented by the following general formulae (1) and (2), respectively:

$$R^1O\text{-}(EO)_mSO_3M \qquad (1)$$

$$R^2O\text{-}(AO)_nSO_3M \qquad (2),$$

wherein $R^1$ and $R^2$ may be the same or different from each other, and are respectively an alkyl group having 8 to 24 carbon atoms; EO is an ethyleneoxy group; AO is an alkyleneoxy group having 3 or 4 carbon atoms; n is a number of more than 0 and not more than 3 ($0 < n \leq 3$); and M is a cation.

JP 61-81496A (Patent Literature 2) discloses a detergent composition containing (A) from about 0.1% to about 1.5% of a water-soluble cellulose thickening agent, (B) from about 0.5% to about 20% of a solvent selected from the group consisting of ethylene glycol, propylene glycol, polyoxyethylene glycol, polyoxypropylene glycol, etc., and a mixture thereof; (C) from about 10% to about 50% of a synthetic surfactant, (D) from about 0.001% to about 1.0% of an electrolyte, and (E) from about 50% to about 80% of water.

SUMMARY OF THE INVENTION

The present invention relates to the following aspects [1] and [2].

[1] A surfactant composition including:

a component (A): a polyol having not less than 2 and not more than 3 carbon atoms;
a component (B): a polyoxyalkylene alkyl or alkenyl ether sulfate or a salt thereof represented by the following general formula (I):

$$R^1O\text{-}(R^2O)_mSO_3M \qquad (I)$$

wherein $R^1$ is an alkyl or alkenyl group having not less than 16 and not more than 24 carbon atoms; $R^2O$ is an oxyalkylene group; m represents an average molar number of addition of an alkyleneoxide, and is a number of not less than 0.5 and not more than 10; and M is a hydrogen atom or a cation;
a component (C): at least one compound selected from the group consisting of an alkali metal salt other than the component (B) and an alkali metal hydroxide; and
a component (D): water,

in which a content of the component (A) in the composition is not less than 25% by mass and not more than 98% by mass,
a content of the component (B) in the composition is not less than 0.05% by mass and not more than 10% by mass in terms of the sulfate,
a total content of alkali metals (X) derived from the polyoxyalkylene alkyl or alkenyl ether sulfate salt as the component (B) and derived from the at least one compound selected from the group consisting of the alkali metal salt and the alkali metal hydroxide as the component (C), in the composition is not more than 2.5% by mass in terms of the metal elements, and
a molar ratio of the alkali metals (X) to the component (B) [(X)/(B)] is not less than 1.5.
[2] A process for producing the surfactant composition as defined in the above aspect [1], including the step of compounding the components (A), (B), (C) and (D).

BRIEF DESCRIPTION OF THE DRAWING

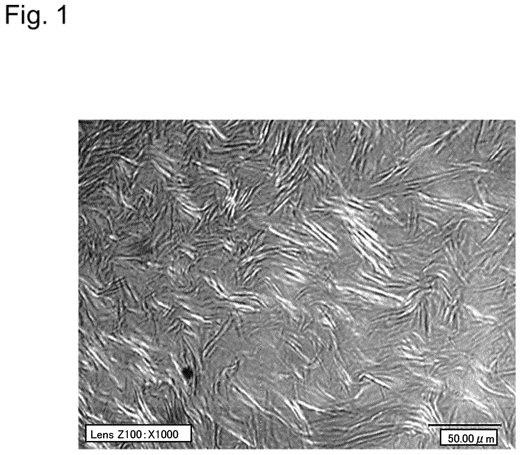

FIG. 1 is a view showing the surfactant composition obtained in Example 13 when observed by a polarization microscope.

DETAILED DESCRIPTION OF THE INVENTION

In the surfactant compositions described in the Patent Literatures 1 and 2, etc., it is required to use a large amount of a salt or a surfactant in order to increase the viscosity of the composition.
The present invention relates to a surfactant composition that is excellent in storage stability (hereinafter also referred to merely as "stability") while maintaining high viscosity thereof even when the concentration of a surfactant used therein is low, and a process for producing the surfactant composition.
The present inventors have found that a surfactant composition including a specific amount of a component (A): a polyol having not less than 2 and not more than 3 carbon atoms; a component (B): a polyoxyalkylene alkyl or alkenyl ether sulfate in which the alkyl or alkenyl group has not less than 16 and not more than 24 carbon atoms, or a salt thereof; a component (C): at least one compound selected from the group consisting of an alkali metal salt other than the component (B) and an alkali metal hydroxide; and a component (D): water is excellent in stability while maintaining high viscosity thereof even when the concentration of a surfactant used therein is low.
That is, the present invention relates to the following aspects [1] and [2].

[1] A surfactant composition including:

a component (A): a polyol having not less than 2 and not more than 3 carbon atoms;
a component (B): a polyoxyalkylene alkyl or alkenyl ether sulfate or a salt thereof represented by the following general formula (I):

$$R^1O\text{-}(R^2O)_mSO_3M \qquad (I)$$

wherein $R^1$ is an alkyl or alkenyl group having not less than 16 and not more than 24 carbon atoms; $R^2O$ is an oxyalkylene group; m represents an average molar number of addition of an alkyleneoxide, and is a number of not less than 0.5 and not more than 10; and M is a hydrogen atom or a cation;
a component (C): at least one compound selected from the group consisting of an alkali metal salt other than the component (B) and an alkali metal hydroxide; and
a component (D): water,

in which a content of the component (A) in the composition is not less than 25% by mass and not more than 98% by mass,
a content of the component (B) in the composition is not less than 0.05% by mass and not more than 10% by mass in terms of the sulfate,
a total content of alkali metals (X) derived from the polyoxyalkylene alkyl or alkenyl ether sulfate salt as the component (B) and derived from the at least one compound selected from the group consisting of the alkali metal salt and the alkali metal hydroxide as the component (C), in the composition is not more than 2.5% by mass in terms of the metal elements, and
a molar ratio of the alkali metals (X) to the component (B) [(X)/(B)] is not less than 1.5.
[2] A process for producing the surfactant composition as defined in the above aspect [1], including the step of compounding the components (A), (B), (C) and (D).

In accordance with the present invention, it is possible to provide a surfactant composition that is excellent in stability while maintaining high viscosity thereof even when the concentration of the surfactant in the composition is lower than that in the conventional surfactant compositions containing an alkyl ether sulfate salt.

[Surfactant Composition]

The surfactant composition according to the present invention includes:

a component (A): a polyol having not less than 2 and not more than 3 carbon atoms;
a component (B): a polyoxyalkylene alkyl or alkenyl ether sulfate or a salt thereof represented by the following general formula (I):

$$R^1O\text{-}(R^2O)_mSO_3M \qquad (I)$$

wherein $R^1$ is an alkyl or alkenyl group having not less than 16 and not more than 24 carbon atoms; $R^2O$ is an oxyalkylene group; m represents an average molar number of addition of an alkyleneoxide, and is a number of not less than 0.5 and not more than 10; and M is a hydrogen atom or a cation;
component (C): at least one compound selected from the group consisting of an alkali metal salt other than the component (B) and an alkali metal hydroxide; and
a component (D): water,

in which a content of the component (A) in the composition is not less than 25% by mass and not more than 98% by mass, a content of the component (B) in the composition is not less than 0.05% by mass and not more than 10% by mass in terms of the sulfate,
a total content of alkali metals (X) derived from the polyoxyalkylene alkyl or alkenyl ether sulfate salt as the component (B) and derived from the at least one compound selected from the group consisting of the alkali metal salt and the alkali metal hydroxide as the component (C), in the composition is not more than 2.5% by mass in terms of the metal elements, and
a molar ratio of the alkali metals (X) to the component (B) [(X)/(B)] is not less than 1.5.

The surfactant composition according to the present invention is excellent in stability while maintaining high viscosity thereof even when the concentration of the surfactant in the composition is lower than that in the conventional surfactant compositions containing an alkyl ether sulfate salt. The reason why the aforementioned advantageous effect can be attained by the present invention is considered as follows though it is not clearly determined yet.

That is, it is considered that by controlling the content of the component (A), the content of the component (B), the total content of the alkali metals (X) which are derived from the polyoxyalkylene alkyl or alkenyl ether sulfate salt as the component (B) and derived from the at least one compound selected from the group consisting of the alkali metal salt and the alkali metal hydroxide as the component (C), and the content of the component (D) in the composition to the respective specific ranges, it becomes possible to form a structural body constituted of the components (A) through (C) and exhibit the effect of increasing viscosity of the composition owing to an interaction between the structural bodies.

In the following, the respective components are described in detail.

<Component (A)>

The surfactant composition of the present invention contains a polyol having not less than 2 and not more than 3 carbon atoms as a component (A).

Examples of the polyol having not less than 2 and not more than 3 carbon atoms include diols such as ethylene glycol, 1,2-propanediol and 1,3-propanediol, and triols such as glycerin. From the viewpoint of improving viscosity of the surfactant composition, among these polyols, preferred are diols. Of these polyols having not less than 2 and not more than 3 carbon atoms, from the viewpoint of improving viscosity of the surfactant composition, preferred is at least one polyol selected from the group consisting of ethylene glycol, 1,2-propanediol and glycerin, more preferred is at least one polyol selected from the group consisting of ethylene glycol and 1,2-propanediol, and even more preferred is ethylene glycol. These polyols may be used alone or in the form of a mixture of any two or more thereof.

<Component (B)>

The surfactant composition of the present invention also contains, as a component (B), a polyoxyalkylene alkyl or alkenyl ether sulfate or a salt thereof represented by the following general formula (I):

$$R^1O\text{-}(R^2O)_mSO_3M \qquad (I)$$

wherein $R^1$ is an alkyl or alkenyl group having not less than 16 and not more than 24 carbon atoms; $R^2O$ is an oxyalkylene group; m represents an average molar number of addition of an alkyleneoxide, and is a number of not less than 0.5 and not more than 10; and M is a hydrogen atom or a cation.

$R^1$ in the general formula (I) is an alkyl or alkenyl group having not less than 16 and not more than 24 carbon atoms, preferably a linear or branched alkyl or alkenyl group, more preferably a linear or branched alkyl group, and even more preferably a linear alkyl group.

The number of carbon atoms in $R^1$ as the alkyl or alkenyl group is not less than 16 and preferably not less than 18 from the viewpoint of increasing viscosity of the resulting surfactant composition, and more preferably not less than 20 and even more preferably not less than 22 from the viewpoint of increasing viscosity of the resulting surfactant composition and improving stability of the surfactant composition. Also, the number of carbon atoms in the alkyl or alkenyl group as $R^1$ is not more than 24 and preferably not more than 22 from the viewpoint of a good availability, and even more preferably 22 from the viewpoint of all the requirements mentioned above.

Specific examples of the alkyl or alkenyl group having not less than 16 and not more than 24 carbon atoms which is represented by $R^1$ include alkyl groups such as a cetyl group, a margaryl group, an isostearyl group, a 2-heptyl undecyl group, a stearyl group, an arachidyl group, a behenyl group and a lignoceryl group; and alkenyl groups such as an oleyl group.

$R^1$ as the alkyl or alkenyl group is preferably at least one group selected from the group consisting of a cetyl group, a margaryl group, an isostearyl group, a 2-heptyl undecyl group, a stearyl group, an arachidyl group, a behenyl group and a lignoceryl group, more preferably at least one group selected from the group consisting of an isostearyl group, a 2-heptyl undecyl group, a stearyl group, an arachidyl group and a behenyl group, and even more preferably a behenyl group.

The alkylene group of the oxyalkylene group represented by $R^2O$ is preferably a linear or branched alkylene group and more preferably a linear alkylene group. The number of carbon atoms in the alkylene group is preferably not less than 2, and is also preferably not more than 4, more preferably not more than 3, and even more preferably 2.

The oxyalkylene group represented by $R^2O$ is preferably at least one group selected from the group consisting of an oxyethylene group, an oxypropylene group, an oxy 1-methyl trimethylene group, an oxy trimethylene group and an oxy tetramethylene group, more preferably at least one group selected from the group consisting of an oxyethylene group and an oxypropylene group, and even more preferably an oxyethylene group.

The symbol m representing an average molar number of addition of an alkyleneoxide is not less than 0.5, preferably not less than 1, more preferably not less than 1.5, even more preferably not less than 2 and further even more preferably not less than 3 from the viewpoint of improving viscosity of the resulting surfactant composition. From the same viewpoint as described above, m is not more than 10, preferably not more than 8, more preferably not more than 7, even more preferably not more than 6, further even more preferably not more than 5 and still further even more preferably 4.

When M in the general formula (I) is a cation, the compound represented by the general formula (I) is in the form of a polyoxyalkylene alkyl or alkenyl ether sulfate salt.

Examples of the cation represented by M in the general formula (I) include an alkali metal ion, an alkali earth metal ion, an ammonium ion and an alkanol ammonium ion such as a triethanol ammonium ion, etc. Examples of the alkali metal include lithium, sodium and potassium. Examples of the alkali earth metal include calcium, etc. M in the general formula (I) is preferably an alkali metal ion, more preferably at least one ion selected from the group consisting of a sodium ion and a potassium ion, and even more preferably a sodium ion. Also, the polyoxyalkylene alkyl or alkenyl ether sulfate or salts thereof may be used alone or in the form of a mixture of any two or more thereof.

<Component (C)>

The surfactant composition of the present invention contains at least one compound selected from the group consisting of an alkali metal salt other than the component (B) and an alkali metal hydroxide as component (C). The term "other than the component (B)" means that the alkali metal salt is other than the aforementioned alkali metal salt as the component (B).

Meanwhile, the component (C) may be previously mixed with the component (B). In this case, there may be used such an embodiment that the alkali metal hydroxide is added in an excessive amount exceeding a neutralization equivalent thereof to the polyoxyalkylene alkyl or alkenyl ether sulfate represented by the aforementioned general formula (I).

The alkali metal contained in the alkali metal salt and the alkali metal hydroxide as the component (C) is preferably at least one alkali metal selected from the group consisting of lithium, sodium and potassium, more preferably at least one alkali metal selected from the group consisting of sodium and potassium and even more preferably potassium, from the viewpoint of improving viscosity of the resulting surfactant composition.

Examples of the alkali metal hydroxide as the component (C) include hydroxides such as sodium hydroxide and potassium hydroxide. Of these compounds, from the viewpoint of improving viscosity of the resulting surfactant composition, preferred is at least one compound selected from the group consisting of sodium hydroxide and potassium hydroxide, and more preferred is potassium hydroxide. These alkali metal hydroxides may be used alone or in the form of a mixture of any two or more thereof.

The alkali metal salt as the component (C) is at least one compound selected from the group consisting of inorganic salts and organic salts other than the aforementioned component (B). Examples of the inorganic salts include nitric acid salts such as sodium nitrate and potassium nitrate; sulfuric acid salts such as sodium sulfate and potassium sulfate; and halogenated salts such as sodium chloride and potassium chloride. Examples of the organic salts other than the aforementioned component (B) include aliphatic carboxylic acid salts such as sodium acetate, potassium acetate, sodium propionate and potassium propionate; aliphatic dicarboxylic acid salts such as disodium succinate, dipotassium succinate, disodium adipate and dipotassium adipate; citric acid salts such as sodium citrate and potassium citrate; and malic acid salts such as disodium malate and dipotassium malate. These alkali metal salts may be used alone or in the form of a mixture of any two or more thereof.

<Component (D)>

The surfactant composition of the present invention also contains water as a component (D).

[Process for Producing Surfactant Composition]

The surfactant composition of the present invention is produced by compounding the components (A), (B), (C) and (D). The compounding order of the respective components is not particularly limited. It is preferred that after all of the components are compounding with each other, the resulting mixture is preferably heated to a temperature not lower than 60°C and more preferably not lower than 80°C, and also preferably not higher than 100°C, if required followed by stirring the mixture, to dissolve all the components therein, and then cooled up to room temperature (20°C).

[Amounts of Respective Components to be Compounded]

The amounts of the respective components to be compounded in the surfactant composition of the present invention are as follows.

The amount of the component (A) to be compounded is preferably not less than 25% by mass, more preferably not less than 30% by mass, even more preferably not less than 40% by mass and further even more preferably not less than 50% by mass on the basis of 100% by mass as a whole amount of the surfactant composition from the viewpoint of improving viscosity of the resulting surfactant composition, and is also preferably not more than 98% by mass, more preferably not more than 90% by mass, even more preferably not more than 80% by mass, further even more preferably not more than 70% by mass and still further even more preferably not more than 60% by mass on the basis of 100% by mass as a whole amount of the surfactant composition from the viewpoint of reducing the production cost and increasing viscosity of the resulting surfactant composition even when using the component (A) in a small amount.

The amount of the component (B) to be compounded is preferably not less than 0.05% by mass, more preferably not less than 0.1% by mass and even more preferably not less than 0.2% by mass in terms of the sulfate on the basis of 100% by mass as a whole amount of the surfactant composition from the viewpoint of improving viscosity of the resulting surfactant composition, and is also preferably not more than 10% by mass, more preferably not more than 7% by mass, even more preferably not more than 5% by mass, further even more preferably not more than 3% by mass, still further even more preferably not more than 1% by mass, still further even more preferably not more than 0.7% by mass, still further even more preferably not more than 0.5% by mass, still further even more preferably not more than 0.4% by mass and still further even more preferably not more than 0.3% by mass in terms of the sulfate on the basis of 100% by mass as a whole amount of the surfactant composition from the viewpoint of reducing the production cost and improving viscosity of the resulting surfactant composition even when the surfactant is used in a small amount. Meanwhile, the amount "in terms of the sulfate" means that the amount of the component (B) to be compounded is calculated in terms of the polyoxyalkylene alkyl or alkenyl ether sulfate represented by the aforementioned general formula (I) (i.e., the compound of the general formula (I) in which M is a hydrogen atom). In addition, in the case where the component (B) is in the form of a mixture, the amount of the component (B) to be compounded is a sum of the amounts of respective components contained in the mixture. The term "amount in terms of a sulfate" which is described hereinunder also has the same meaning as defined above.

The amount of the component (C) to be compounded is preferably not less than 0.07% by mass, more preferably not less than 0.15% by mass, even more preferably not less than 0.2% by mass, further even more preferably not less than 0.4% by mass, still further even more preferably not less than 0.7% by mass and still further even more preferably not less than 0.9% by mass on the basis of 100% by mass as a whole amount of the surfactant composition from the viewpoint of improving viscosity of the resulting surfactant composition, and is also preferably not more than 3.5% by mass, more preferably not more than 2.5% by mass, even more preferably not more than 2% by mass, further even more preferably not more than 1.8% by mass, still further even more preferably not more than 1.5% by mass and still further even more preferably not more than 1.3% by mass on the basis of 100% by mass as a whole amount of the surfactant composition from the same viewpoint as described above.

The amount of the component (D) to be compounded is preferably not less than 2% by mass, more preferably not less than 5% by mass, even more preferably not less than 10% by mass, further even more preferably not less than 15% by mass, still further even more preferably not less than 20% by mass, still further even more preferably not less than 30% by mass and still further even more preferably not less than 40% by mass on the basis of 100% by mass as a whole amount of the surfactant composition from the viewpoint of reducing the amount of the component (A) used and reducing the production cost, and is also preferably not more than 72% by mass, more preferably not more than 70% by mass, even more preferably not more than 60% by mass, further even more preferably not more than 55% by mass and still further even more preferably not more than 50% by mass on the basis of 100% by mass as a whole amount of the surfactant composition from the viewpoint of improving viscosity of the resulting surfactant composition.

[Contents of Respective Components in Surfactant Composition]

The contents of the respective components in the surfactant composition of the present invention are as follows.

The content of the component (A) in the surfactant composition of the present invention is not less than 25% by mass, preferably not less than 30% by mass, more preferably not less than 40% by mass and even more preferably not less than 50% by mass from the viewpoint of improving viscosity of the resulting surfactant composition, and is also not more than 98% by mass, preferably not more than 90% by mass, more preferably not more than 80% by mass, even more preferably not more than 70% by mass and further even more preferably not more than 60% by mass from the viewpoint of reducing the production cost and increasing viscosity of the resulting surfactant composition even when using the component (A) in a small amount.

The content of the component (B) in the surfactant composition of the present invention is not less than 0.05% by mass, preferably not less than 0.1% by mass and more preferably not less than 0.2% by mass in terms of the sulfate from the viewpoint of improving viscosity of the resulting surfactant composition, and is also not more than 10% by mass, preferably not more than 7% by mass, more preferably not more than 5% by mass, even more preferably not more than 3% by mass, further even more preferably not more than 1% by mass, still further even more preferably not more than 0.7% by mass, still further even more preferably not more than 0.5% by mass, still further even more preferably not more than 0.4% by mass and still further even more preferably not more than 0.3% by mass in terms of the sulfate from the viewpoint of reducing the production cost and improving viscosity of the resulting surfactant composition even when using the surfactant in a small amount.

The total content of the alkali metals (X) derived from the polyoxyalkylene alkyl or alkenyl ether sulfate salt as the aforementioned component (B) and derived from the at least one compound selected from the group consisting of the alkali metal salt and the alkali metal hydroxide as the aforementioned component (C) in the surfactant composition of the present invention is preferably not less than 0.05% by mass, more preferably not less than 0.1% by mass, even more preferably not less than 0.15% by mass, further even more preferably not less than 0.3% by mass, still further even more preferably not less than 0.5% by mass, still further even more preferably not less than 0.8% by mass and still further even more preferably not less than 1.0% by mass in terms of the metal elements from the viewpoint of improving viscosity of the resulting surfactant composition, and is also not more than 2.5% by mass, preferably not more than 2% by mass, more preferably not more than 1.5% by mass, even more preferably not more than 1.3% by mass and further even more preferably not more than 1.2% by mass in terms of the metal elements from the same viewpoint as described above.

Meanwhile, the respective amounts of the aforementioned alkali metals (X) in terms of the metal elements are determined according to the following formula:

$$\text{Amount compounded} \times (\text{atomic weight of the alkali metal in the compound/molecular weight of the compound}).$$

For example, in the case of using sodium hydroxide, the amount of the alkali metal (X) in terms of the metal element can be determined from the calculation formula: amount compounded $\times$ (23/40). Also, in the case of using potassium chloride, the amount of the alkali metal (X) in terms of the metal element can be determined from the calculation formula: amount compounded $\times$ (39/74.5). Alternatively, the contents of the respective alkali metals (X) in the composition may be directly determined by ICP emission spectrometry, etc. The total content of the alkali metals (X) in the surfactant composition in the case where the composition contains a plurality of alkali metals may be determined from a sum of the amounts of the respective alkali metals in terms of the metal elements.

The content of the component (C) in the surfactant composition of the present invention is preferably not less than 0.07% by mass, more preferably not less than 0.15% by mass, even more preferably not less than 0.2% by mass, further even more preferably not less than 0.4% by mass, still further even more preferably not less than 0.7% by mass and still further even more preferably not less than 0.9% by mass in terms of the metal element from the viewpoint of improving viscosity of the resulting surfactant composition, and is also preferably not more than 3.5% by mass, more preferably not more than 2.5% by mass, even more preferably not more than 2% by mass, further even more preferably not more than 1.8% by mass, still further even more preferably not more than 1.5% by mass and still further even more preferably not more than 1.3% by mass in terms of the metal element from the same viewpoint as described above.

The content of the component (D) in the surfactant composition of the present invention is preferably not less than 2% by mass, more preferably not less than 5% by mass, even more preferably not less than 10% by mass, further even more preferably not less than 15% by mass, still further even more preferably not less than 20% by mass, still further even more preferably not less than 30% by mass and still further even more preferably not less than 40% by mass from the viewpoint of reducing the amount of the component (A) used and reducing the production cost, and is also preferably not more than 72% by mass, more preferably not more than 70% by mass, even more preferably not more than 60% by mass, further even more preferably not more than 55% by mass and still further even more preferably not more than 50% by mass from the viewpoint of improving viscosity of the resulting surfactant composition.

The mass ratio of the component (A) to the component (B) [(A)/(B)] in the surfactant composition of the present invention is preferably not more than 1,900, more preferably not more than 1,500, even more preferably not more than 1,000, further even more preferably not more than 800, still further even more preferably not more than 500, still further even more preferably not more than 400 and still further even more preferably not more than 300 from the viewpoint of improving viscosity of the resulting surfactant composition, and is also preferably not less than 2.5, more preferably not less than 5, even more preferably not less than 10, further even more preferably not less than 20, still further even more preferably not less than 50, still further even more preferably not less than 100 and still further even more preferably not less than 200 from the viewpoint of increasing viscosity of the resulting surfactant composition even when using the surfactant in a smaller amount. Meanwhile, the mass of the component (B) is such an amount as calculated in terms of the sulfate.

The mass ratio of the component (A) to the alkali metals (X) [(A)/(X)] in the surfactant composition of the present invention is preferably not more than 1,000, more preferably not more than 700, even more preferably not more than 500, further even more preferably not more than 400, still further even more preferably not more than 300, still further even more preferably not more than 200 and still further even more preferably not more than 100 from the viewpoint of improving viscosity of the resulting surfactant composition, and is also preferably not less than 20, more preferably not less than 30 and even more preferably not less than 40 from the same viewpoint as described above.

The mass ratio of the component (A) to the component (D) [(A)/(D)] in the surfactant composition of the present invention is preferably not more than 45, more preferably not more than 30, even more preferably not more than 20, further even more preferably not more than 10, still further even more preferably not more than 7, still further even more preferably not more than 5, still further even more preferably not more than 4 and still further even more preferably not more than 3 from the viewpoint of increasing viscosity of the resulting surfactant composition even when using the surfactant composition in a smaller amount, and is also preferably not less than 0.35, more preferably not less than 0.5, even more preferably not less than 0.7, further even more preferably not less than 1 and still further even more preferably not less than 1.3 from the viewpoint of improving viscosity of the resulting surfactant composition.

The total content of the component (A) and the component (D) in the surfactant composition of the present invention is preferably not more than 99.6% by mass, more preferably not more than 99.0% by mass, even more preferably not more than 98.5% by mass and further even more preferably not more than 98.3% by mass from the viewpoint of improving viscosity of the resulting surfactant composition by compounding the other components therein, and is also preferably not less than 85.0% by mass, more preferably not less than 88.0% by mass, even more preferably not less than 90.0% by mass, further even more preferably not less than 93.0% by mass, still further even more preferably not less than 95.0% by mass, still further even more preferably not less than 97.0% by mass and still further even more preferably not less than 98.0% by mass from the viewpoint of increasing viscosity of the resulting surfactant composition even when using the surfactant in a smaller amount.

The mass ratio of the component (B) to the alkali metals (X) [(B)/(X)] in the surfactant composition of the present invention is preferably not less than 0.02, more preferably not less than 0.05 and even more preferably not less than 0.1 from the viewpoint of improving viscosity of the resulting surfactant composition, and is also preferably not more than 50, more preferably not more than 20, even more preferably not more than 5, further even more preferably not more than 3, still further even more preferably not more than 1 and still further even more preferably not more than 0.5 from the viewpoint of increasing viscosity of the resulting surfactant composition even when using the surfactant in a smaller amount. Meanwhile, the mass of the component (B) is such an amount as calculated in terms of the sulfate.

The molar ratio of the alkali metals (X) to the component (B) to [(X)/(B)] in the surfactant composition of the present invention is preferably not less than 1.5, more preferably not less than 2.5, even more preferably not less than 3, further even more preferably not less than 5, still further even more preferably not less than 10, still further even more preferably not less than 20, still further even more preferably not less than 30 and still further even more preferably not less than 40 from the viewpoint of improving viscosity of the resulting surfactant composition, and is also preferably not more than 3,000, more preferably not more than 2,500, even more preferably not more than 2,000, further even more preferably not more than 1,500, still further even more preferably not more than 1,000, still further even more preferably not more than 700, still further even more preferably not more than 500, still further even more preferably not more than 400, still further even more preferably not more than 300, still further even more preferably not more than 200, still further even more preferably not more than 100 and still further even more preferably not more than 80 from the same viewpoint as described above. Meanwhile, the number of moles of each of the alkali metals (X) is the numeral value obtained by dividing the amount of the alkali metal in terms of the metal element by an atomic weight of the metal element (a relative mass of the metal element assuming that a mass of $^{12}$C is 12), and when a plurality of alkali metals are present, the aforementioned value means a total number of moles of the respective alkali metals. The number of moles of the component (B) is the numeral value obtained by dividing a mass of the component (B) (as an amount in terms of the sulfate) by a molecular weight of the polyoxyalkylene alkyl or alkenyl ether sulfate represented by the aforementioned general formula (I), and when the component (B) is in the form of a mixture, the number of moles of the component (B) means a total number of moles of respective components in the mixture.

The properties of the surfactant composition of the present invention are as follows.

The viscosity of the surfactant composition of the present invention as measured at an ordinary temperature (25°C) is preferably not less than 6 mPa•s, more preferably not less than 10 mPa•s, even more preferably not less than 15 mPa•s, further even more preferably not less than 20 mPa•s and still further even more preferably not less than 30 mPa•s, and is also preferably not more than 300 mPa•s, more preferably not more than 200 mPa••s, even more preferably not more than 100 mPa•s and further even more preferably not more than 50 mPa•s. The aforementioned viscosity is the value measured by the method described in Examples below.

The pH value of the surfactant composition of the present invention as measured at a temperature of 20°C is preferably not less than 6 and more preferably not less than 7, and is also preferably not more than 12 and more preferably not more than 11.

The thus obtained surfactant composition of the present invention may be further compounded with the other components such as, for example, drugs, powders and antiseptic agents, and may be suitably used, for example, in the applications including cosmetics, detergents, coating materials, inks, lubricants, adhesives, agricultural compounding agents, drilling fluids for petroleum, etc.

With respect to the aforementioned embodiments, the present invention further provides the following aspects relating to the surfactant composition and the process for producing the surfactant composition.

<1> A surfactant composition including:

a component (A): a polyol having not less than 2 and not more than 3 carbon atoms;
a component (B): a polyoxyalkylene alkyl or alkenyl ether sulfate or a salt thereof represented by the following general formula (I):

$$R^1O\text{-}(R^2O)_mSO_3M \qquad (I)$$

wherein $R^1$ is an alkyl or alkenyl group having not less than 16 and not more than 24 carbon atoms; $R^2O$ is an oxyalkylene group; m represents an average molar number of addition of an alkyleneoxide, and is a number of not less than 0.5 and not more than 10; and M is a hydrogen atom or a cation;
a component (C): at least one compound selected from the group consisting of an alkali metal salt other than the component (B) and an alkali metal hydroxide; and
a component (D): water,

in which a content of the component (A) in the composition is not less than 25% by mass and not more than 98% by mass,
a content of the component (B) in the composition is not less than 0.05% by mass and not more than 10% by mass in terms of the sulfate,
a total content of alkali metals (X) derived from the polyoxyalkylene alkyl or alkenyl ether sulfate salt as the component (B) and derived from the at least one compound selected from the group consisting of the alkali metal salt and the alkali metal hydroxide as the component (C), in the composition is not more than 2.5% by mass in terms of the metal elements, and
a molar ratio of the alkali metals (X) to the component (B) [(X)/(B)] is not less than 1.5.
<2> The surfactant composition according to the aspect <1>, wherein the polyol having not less than 2 and not more than 3 carbon atoms is preferably at least one polyol selected from the group consisting of ethylene glycol, 1,2-propanediol and glycerin, more preferably at least one polyol selected from the group consisting of ethylene glycol and 1,2-propanediol, and even more preferably ethylene glycol.

<3> The surfactant composition according to the aspect <1> or <2>, wherein a number of carbon atoms in $R^1$ in the aforementioned formula (I) is preferably not less than 18, more preferably not less than 20 and even more preferably not less than 22, is also preferably not more than 22 and more preferably 22.

<4> The surfactant composition according to any one of the aspects <1> to <3>, wherein $R^1$ in the aforementioned formula (I) is preferably at least one group selected from the group consisting of a cetyl group, a margaryl group, an isostearyl group, a 2-heptyl undecyl group, a stearyl group, an arachidyl group, a behenyl group and a lignoceryl group, more preferably at least one group selected from the group consisting of an isostearyl group, a 2-heptyl undecyl group, a stearyl group, an arachidyl group and a behenyl group, and even more preferably a behenyl group.

<5> The surfactant composition according to any one of the aspects <1> to <4>, wherein the oxyalkylene group represented by $R^2O$ is preferably at least one group selected from the group consisting of an oxyethylene group, an oxypropylene group, an oxy 1-methyl trimethylene group, an oxytrimethylene group and an oxytetramethylene group, more preferably at least one group selected from the group consisting of an oxyethylene group and an oxypropylene group, and even more preferably an oxyethylene group.

<6> The surfactant composition according to any one of the aspects <1> to <5>, wherein m representing an average molar number of addition of an alkyleneoxide is preferably not less than 1, more preferably not less than 1.5, even more preferably not less than 2 and further even more preferably not less than 3, and is also preferably not more than 8, more preferably not more than 7, even more preferably not more than 6, further even more preferably not more than 5, and still further even more preferably 4.

<7> The surfactant composition according to any one of the aspects <1> to <6>, wherein the cation represented by M in the aforementioned formula (I) is preferably an alkali metal ion, more preferably at least one ion selected from the group consisting of a sodium ion and a potassium ion, and even more preferably a sodium ion.

<8> The surfactant composition according to any one of the aspects <1> to <7>, wherein the alkali metal contained in the alkali metal salt and the alkali metal hydroxide as the component (C) is preferably at least one alkali metal selected from the group consisting of lithium, sodium and potassium, more preferably at least one alkali metal selected from the group consisting of sodium and potassium, and even more preferably potassium.

<9> The surfactant composition according to any one of the aspects <1> to <7>, wherein the alkali metal hydroxide as the component (C) is preferably at least one compound selected from the group consisting of sodium hydroxide and potassium hydroxide, and more preferably potassium hydroxide.

<10> The surfactant composition according to any one of the aspects <1> to <9>, wherein the alkali metal salt as the component (C) is at least one compound selected from the group consisting of inorganic acid salts and organic acid salts other than the aforementioned component (B).

<11> The surfactant composition according to any one of the aspects <1> to <10>, wherein an amount of the component (A) to be compounded is preferably not less than 25% by mass, more preferably not less than 30% by mass, even more preferably not less than 40% by mass and further even more preferably not less than 50% by mass, and is also preferably not more than 98% by mass, more preferably not more than 90% by mass, even more preferably not more than 80% by mass, further even more preferably not more than 70% by mass and still further even more preferably not more than 60% by mass, on the basis of 100% by mass as a whole amount of the surfactant composition.

<12> The surfactant composition according to any one of the aspects <1> to <11>, wherein an amount of the component (B) to be compounded is preferably not less than 0.05% by mass, more preferably not less than 0.1% by mass and even more preferably not less than 0.2% by mass, and is also preferably not more than 10% by mass, more preferably not more than 7% by mass, even more preferably not more than 5% by mass, further even more preferably not more than 3% by mass, still further even more preferably not more than 1% by mass, still further even more preferably not more than 0.7% by mass, still further even more preferably not more than 0.5% by mass, still further even more preferably not more than 0.4% by mass and still further even more preferably not more than 0.3% by mass, in terms of the sulfate on the basis of 100% by mass as a whole amount of the surfactant composition.

<13> The surfactant composition according to any one of the aspects <1> to <12>, wherein an amount of the component (C) to be compounded is preferably not less than 0.07% by mass, more preferably not less than 0.15% by mass, even more preferably not less than 0.2% by mass, further even more preferably not less than 0.4% by mass, still further even more preferably not less than 0.7% by mass and still further even more preferably not less than 0.9% by mass, and is also preferably not more than 3.5% by mass, more preferably not more than 2.5% by mass, even more preferably not more than 2% by mass, further even more preferably not more than 1.8% by mass, still further even more preferably not more than 1.5% by mass and still further even more preferably not more than 1.3% by mass on the basis of 100% by mass as a whole amount of the surfactant composition.

<14> The surfactant composition according to any one of the aspects <1> to <13>, wherein an amount of the component (D) to be compounded is preferably not less than 2% by mass, more preferably not less than 5% by mass, even more preferably not less than 10% by mass, further even more preferably not less than 15% by mass, still further even more preferably not less than 20% by mass, still further even more preferably not less than 30% by mass and still further even more preferably not less than 40% by mass, and is also preferably not more than 72% by mass, more preferably not more than 70% by mass, even more preferably not more than 60% by mass, further even more preferably not more than 55% by mass and still further even more preferably not more than 50% by mass, on the basis of 100% by mass as a whole amount of the surfactant composition.

<15> The surfactant composition according to any one of the aspects <1> to <14>, wherein a content of the component (A) in the surfactant composition is preferably not less than 30% by mass, more preferably not less than 40% by mass and even more preferably not less than 50% by mass, and is also preferably not more than 90% by mass, more preferably not more than 80% by mass, even more preferably not more than 70% by mass and further even more preferably not more than 60% by mass.

<16> The surfactant composition according to any one of the aspects <1> to <15>, wherein a content of the component (B) in the surfactant composition is preferably not less than 0.1% by mass and more preferably not less than 0.2% by mass, and is also preferably not more than 7% by mass, more preferably not more than 5% by mass, even more preferably not more than 3% by mass, further even more preferably not more than 1% by mass, still further even more preferably not more than 0.7% by mass, still further even more preferably not more than 0.5% by mass, still further even more preferably not more than 0.4% by mass and still further even more preferably not more than 0.3% by mass, in terms of the sulfate.

<17> The surfactant composition according to any one of the aspects <1> to <16>, wherein a total content of the alkali metals (X) derived from the polyoxyalkylene alkyl or alkenyl ether sulfate salt as the aforementioned component (B) and derived from the at least one compound selected from the group consisting of the alkali metal salt and the alkali metal hydroxide as the aforementioned component (C) in the surfactant composition is preferably not less than 0.05% by mass, more preferably not less than 0.1% by mass, even more preferably not less than 0.15% by mass, further even more preferably not less than 0.3% by mass, still further even more preferably not less than 0.5% by mass, still further even more preferably not less than 0.8% by mass and still further even more preferably not less than 1.0% by mass, and is also preferably not more than 2% by mass, more preferably not more than 1.5% by mass, even more preferably not more than 1.3% by mass and further even more preferably not more than 1.2% by mass, in terms of the metal elements.

<18> The surfactant composition according to any one of the aspects <1> to <17>, wherein a content of the component (C) in the surfactant composition is preferably not less than 0.07% by mass, more preferably not less than 0.15% by mass, even more preferably not less than 0.2% by mass, further even more preferably not less than 0.4% by mass, still further even more preferably not less than 0.7% by mass and still further even more preferably not less than 0.9% by mass, and is also preferably not more than 3.5% by mass, more preferably not more than 2.5% by mass, even more preferably not more than 2% by mass, further even more preferably not more than 1.8% by mass, still further even more preferably not more than 1.5% by mass and still further even more preferably not more than 1.3% by mass, in terms of the metal element.

<19> The surfactant composition according to any one of the aspects <1> to <18>, wherein a content of the component (D) in the surfactant composition is preferably not less than 2% by mass, more preferably not less than 5% by mass, even more preferably not less than 10% by mass, further even more preferably not less than 15% by mass, still further even more preferably not less than 20% by mass, still further even more preferably not less than 30% by mass and still further even more preferably not less than 40% by mass, and is also preferably not more than 72% by mass, more preferably not more than 70% by mass, even more preferably not more than 60% by mass, further even more preferably not more than 55% by mass and still further even more preferably not more than 50% by mass.

<20> The surfactant composition according to any one of the aspects <1> to <19>, wherein a mass ratio of the component (A) to the component (B) [(A)/(B)] in the surfactant composition is preferably not more than 1,900, more preferably not more than 1,500, even more preferably not more than 1,000, further even more preferably not more than 800, still further even more preferably not more than 500, still further even more preferably not more than 400 and still further even more preferably not more than 300, and is also preferably not less than 2.5, more preferably not less than 5, even more preferably not less than 10, further even more preferably not less than 20, still further even more preferably not less than 50, still further even more preferably not less than 100 and still further even more preferably not less than 200.

<21> The surfactant composition according to any one of the aspects <1> to <20>, wherein a mass ratio of the component (A) to the alkali metals (X) [(A)/(X)] in the surfactant composition is preferably not more than 1,000, more preferably not more than 700, even more preferably not more than 500, further even more preferably not more than 400, still further even more preferably not more than 300, still further even more preferably not more than 200 and still further even more preferably not more than 100, and is also preferably not less than 20, more preferably not less than 30 and even more preferably not less than 40.

<22> The surfactant composition according to any one of the aspects <1> to <21>, wherein a mass ratio of the component (A) to the component (D) [(A)/(D)] in the surfactant composition is preferably not more than 45, more preferably not more than 30, even more preferably not more than 20, further even more preferably not more than 10, still further even more preferably not more than 7, still further even more preferably not more than 5, still further even more preferably not more than 4 and still further even more preferably not more than 3, and is also preferably not less than 0.35, more preferably not less than 0.5, even more preferably not less than 0.7, further even more preferably not less than 1 and still further even more preferably not less than 1.3.

<23> The surfactant composition according to any one of the aspects <1> to <22>, wherein a total content of the component (A) and the component (D) in the surfactant composition is preferably not more than 99.6% by mass, more preferably not more than 99.0% by mass, even more preferably not more than 98.5% by mass and further even more preferably not more than 98.3% by mass, and is also preferably not less than 85.0% by mass, more preferably not less than 88.0% by mass, even more preferably not less than 90.0% by mass, further even more preferably not less than 93.0% by mass, still further even more preferably not less than 95.0% by mass, still further even more preferably not less than 97.0% by mass and still further even more preferably not less than 98.0% by mass.

<24> The surfactant composition according to any one of the aspects <1> to <23>, wherein a mass ratio of the component (B) to the alkali metals (X) [(B)/(X)] in the surfactant composition is preferably not less than 0.02, more preferably not less than 0.05 and even more preferably not less than 0.1, and is also preferably not more than 50, more preferably not more than 20, even more preferably not more than 5, further even more preferably not more than 3, still further even more preferably not more than 1 and still further even more preferably not more than 0.5.

<25> The surfactant composition according to any one of the aspects <1> to <24>, wherein a molar ratio of the alkali metals (X) to the component (B) [(X)/(B)] in the surfactant composition is preferably not less than 2.5, more preferably not less than 3, even more preferably not less than 5, further even more preferably not less than 10, still further even more preferably not less than 20, still further even more preferably not less than 30 and still further even more preferably not less than 40, and is also preferably not more than 3,000, more preferably not more than 2,500, even more preferably not more than 2,000, further even more preferably not more than 1,500, still further even more preferably not more than 1,000, still further even more preferably not more than 700, still further even more preferably not more than 500, still further even more preferably not more than 400, still further even more preferably not more than 300, still further even more preferably not more than 200, still further even more preferably not more than 100 and still further even more preferably not more than 80.

<26> The surfactant composition according to any one of the aspects <1> to <25>, wherein a viscosity of the surfactant composition as measured at an ordinary temperature (25°C) is preferably not less than 6 mPa•s, more preferably not less than 10 mPa•s, even more preferably not less than 15 mPa•s, further even more preferably not less than 20 mPa•s and still further even more preferably not less than 30 mPa•s, and is also preferably not more than 300 mPa•s, more preferably not more than 200 mPa•s, even more preferably not more than 100 mPa•s and further even more preferably not more than 50 mPa•s.

<27> The surfactant composition according to any one of the aspects <1> to <26>, wherein the composition is produced by compounding the components (A), (B), (C) and (D).

<28> A process for producing the surfactant composition according to any one of the aspects <1> to <27>, including the step of compounding the components (A), (B), (C) and (D).

EXAMPLES

The surfactant compositions produced in the respective Examples and Comparative Examples were evaluated according to the following methods.

In the following Examples and Comparative Examples, the term "%" indicates "% by mass", unless otherwise specified.

(1) Viscosity

The surfactant composition produced in the respective Examples and Comparative Examples was allowed to stand in a constant-temperature bath controlled to a measuring temperature for 1 hour, and then the viscosity of the surfactant composition was measured using a rheometer "MCR-302 Model" available from Anton Paar GmbH equipped with an attachment "CP50-1" at a rotating speed of 100/s (measuring time: 5 seconds; 25°C).

(2) Appearance

A 50 cc screw vial was filled with 40 g of the surfactant composition produced in the respective Examples and Comparative Examples and hermetically sealed with a cap. The surfactant composition thus filled was allowed to stand at 20°C in a thermostatic chamber capable of being controlled to a constant inside temperature for 1 day, and then observed by naked eyes to examine the condition thereof. The appearance of the surfactant composition was evaluated according to the following ratings.

[Evaluation Ratings]

3: Entirely uniform thick-milky white turbidity occurred.
2: Separated into layers, but no precipitation occurred.
1: Precipitation occurred.
0: Transparent

(3) Storage Stability

A 50 mL sampling bottle was filled with 40 g of the surfactant composition produced in the respective Examples and Comparative Examples and hermetically sealed with a cap. The surfactant composition thus filled was allowed to stand at 20°C in a thermostatic chamber capable of being controlled to a constant inside temperature, and then observed by naked eyes after the elapse of one week and 4 weeks to examine the condition thereof. The storage stability of the surfactant composition was evaluated according to the following ratings.

[Evaluation Ratings]

3: Entirely uniform thick milky white turbidity occurred.
2: Separated into layers, but no precipitation occurred.
1: Precipitation occurred.
0: Transparent

[Production of Polyoxyalkylene Alkyl Ether Sulfate Salt]

Synthesis Method 1

Using a film-type sulfation reactor equipped with an external jacket, an ethoxylate (average molar number of addition of ethyleneoxide: 3.0) of a higher alcohol mainly having 12 carbon atoms ("KALKOL 2098" (tradename) available from Kao Corporation) was flowed down in the form of a thin film at a feed rate of 5.0 L/h, and a sulfur trioxide gas diluted with dried air (sulfur trioxide gas concentration: 1.1% by volume) was added to the reactor at a reactor cooling temperature of 40°C at a feed rate of 130 L/min (molar ratio of sulfur trioxide/ethoxylate: 1.00) to subject the ethoxylate to sulfation reaction.

The thus obtained polyoxyethylene alkyl ether sulfate was neutralized with a 2.5% sodium hydroxide aqueous solution (molar ratio of sodium hydroxide/polyoxyethylene alkyl ether sulfate: 1.10) so as to obtain an aqueous solution of a polyoxyethylene alkyl ether sulfate sodium salt [component (B)] with a concentration of not less than 23% and not more than 27%, thereby synthesizing the polyoxyethylene alkyl ether sulfate sodium salt having 12 carbon atoms (average molar number of addition of ethyleneoxide: 3.0).

The same method as defined above was repeated except for using an ethoxylate (average molar number of addition of ethyleneoxide: 4.0) of a higher alcohol mainly having 16 carbon atoms ("KALKOL 6098" (tradename) available from Kao Corporation) and an ethoxylate (average molar number of addition of ethyleneoxide: 1.5, 4.0) of a higher alcohol mainly having 18 carbon atoms ("KALKOL 8098" (tradename) available from Kao Corporation), thereby synthesizing polyoxyethylene alkyl ether sulfate sodium salts of the respective ethoxylates.

Synthesis Example 2

Two hundred eleven grams (211 g) of an ethoxylate (average molar number of addition of ethyleneoxide: 4.0) of a higher alcohol mainly having 22 carbon atoms ("KALKOL 220-80" (tradename) available from Kao Corporation) were subjected to sulfation reaction with sulfamic acid (molar ratio of sulfamic acid/ethoxylate: 1.10) at a temperature of 110°C.

Two hundred fifty six grams (256 g) of the thus obtained polyoxyethylene alkyl ether sulfate was neutralized with 1,280 mL of a 1.59% sodium hydroxide aqueous solution (molar ratio of sodium hydroxide/polyoxyethylene alkyl ether sulfate: 1.10). Then, the resulting neutralization reaction mixture was heated at 80°C under reduced pressure to remove ammonia therefrom (deammoniation). The obtained reaction mixture was mixed with ion-exchanged water such that the concentration of a polyoxyethylene alkyl ether sulfate sodium salt therein was controlled to not less than 8% and not more than 13%, thereby synthesizing the polyoxyethylene alkyl ether sulfate sodium salt having 22 carbon atoms (average molar number of addition of ethyleneoxide: 4.0).

Examples 1 to 23 and Comparative Examples 1 to 18

The respective components were charged in formulation and compounding amounts shown in Tables 1 to 6 into a 50 mL sampling bottle, and the contents of the sampling bottle were allowed to stand at 90°C for 1 hour to thereby completely dissolve the components therein. Thereafter, the resulting reaction solution was cooled to room temperature (20°C) to produce a surfactant composition. The thus obtained surfactant composition was evaluated with respect to viscosity, appearance and storage stability after the elapse of 1 week and 4 weeks by the aforementioned evaluation methods. The results are shown in Tables 1 to 6.

In addition, the surfactant composition obtained in Example 13 was observed by a polarization microscope. The observation results are shown in FIG. 1.

Meanwhile, the details of the respective components shown in Tables 1 to 6 are as follows.

[Component (A)]

- Ethylene glycol: "WAKO 1st Grade Reagent" available from Wako Pure Chemical Industries, Ltd.; purity: > 99.0%
- 1,2-Propanediol: "WAKO 1st Grade Reagent" available from Wako Pure Chemical Industries, Ltd.; purity: > 98.0%
- Glycerin: "WAKO Special Grade Reagent" available from Wako Pure Chemical Industries, Ltd.; purity: > 99.0%

[Component (B)]

- C16ESNa (EO = 4): Polyoxyethylene alkyl ether sulfate sodium salt having 16 carbon atoms (average molar number of addition of ethyleneoxide: 4.0) (molar ratio: 1.1).
- C18ESNa (EO = 4): Polyoxyethylene alkyl ether sulfate sodium salt having 18 carbon atoms (average molar number of addition of ethyleneoxide: 4.0) (molar ratio: 1.1).
- C18ESNa (EO = 1.5): Polyoxyethylene alkyl ether sulfate sodium salt having 18 carbon atoms (average molar number of addition of ethyleneoxide: 1.5) (molar ratio: 1.1).
- C22ESNa (EO = 4): Polyoxyethylene alkyl ether sulfate sodium salt having 22 carbon atoms (average molar number of addition of ethyleneoxide: 4.0) (molar ratio: 1.1).

[Component (C)]

- KOH: Potassium hydroxide (solid content: 100%)
- NaOH: Sodium hydroxide (solid content: 100%)
- KCL: Potassium chloride (solid content: 100%)

[Other Components]

- Ethanol: "WAKO 1st Grade Reagent" available from Wako Pure Chemical Industries, Ltd.; purity: > 99.5%
- C12ESNa (EO = 3): Polyoxyethylene alkyl ether sulfate sodium salt having 12 carbon atoms (average molar number of addition of ethyleneoxide: 3.0) (molar ratio: 1.1).
- $Ca(OH)_2$: Calcium hydroxide "WAKO 1st Grade Reagent" available from Wako Pure Chemical Industries, Ltd.; purity: > 85%
- Triethanol amine: Available from Kishida Chemical Co., Ltd.; purity: 97%

The content (in terms of a sulfate) of the component (B) as shown in Tables 1 to 6, for example, the content (in terms of a sulfate) of C22ESNa (EO = 4) which represents $C_{22}H_{45}O(EO)_4SO_3H$ (molecular weight: 582), is calculated by multiplying a compounding amount thereof by a mass ratio [= 582/607.3].

The content (in terms of metal elements) of the alkali metals (X) as shown in Tables 1 to 6 is a total content of the alkali metal derived from the aforementioned polyoxyalkylene alkyl ether sulfate salt and the alkali metal derived from the at least one compound selected from the group consisting of the aforementioned alkali metal salt and alkali metal hydroxide. For example, in the case where potassium hydroxide and C22ESNa (EO = 4) are used as the alkali metal hydroxide and the polyoxyalkylene alkyl ether sulfate salt, respectively, the content (in terms of metal elements) of the alkali metals (X) is calculated as a total content of potassium of the potassium hydroxide and sodium of C22ESNa (EO = 4).

The molar ratio of the alkali metals (X) to the component (B) [(X)/(B)] as shown in Tables 1 to 6 is calculated by determining the numbers of moles of the respective components as described hereinunder.

The number of moles of the alkali metals (X) is calculated as follows. For example, in the case where potassium hydroxide and C22ESNa (EO = 4) are used as the alkali metal hydroxide and the polyoxyalkylene alkyl ether sulfate salt, respectively, the number of moles of the respective alkali metals is determined from the content thereof in the surfactant composition of the present invention, and the number of moles of the alkali metals (X) is calculated as a total number of moles of these alkali metals.

The number of moles of the component (B) is calculated by dividing the content (in terms of a sulfate) of the component (B) in the surfactant composition of the present invention by a molecular weight of the sulfate.

TABLE 1

| | | | Examples | | | | | | | Comparative Examples | |
|---|---|---|---|---|---|---|---|---|---|---|---|
| | | | 1 | 2 | 3 | 4 | 5 | 6 | 7 | 1 | 2 |
| Compounding amounts of respective components (%) | (A) | Ethylene glycol | 27.1 | 45.5 | 52.1 | 58.6 | 76.1 | 82.2 | 96.0 | 0.00 | 19.7 |
| | (B) | C22ESNa (EO = 4)*3 | 0.25 | 0.25 | 0.25 | 0.25 | 0.25 | 0.25 | 0.25 | 0.25 | 0.25 |
| | (C) | KOH*3 | 1.5 | 1.5 | 1.5 | 1.5 | 1.5 | 1.5 | 1.5 | 1.5 | 1.5 |
| | (D) | Water | 71.15 | 52.75 | 46.15 | 39.65 | 22.15 | 16.05 | 2.25 | 98.25 | 78.55 |
| | Total | | 100 | 100 | 100 | 100 | 100 | 100 | 100 | 100 | 100 |
| | (A) + (D) | | 98.25 | 98.25 | 98.25 | 98.25 | 98.25 | 98.25 | 98.25 | 98.25 | 98.25 |
| Contents of respective components (%) | (A) | | 27.10 | 45.50 | 52.10 | 58.60 | 76.10 | 82.20 | 96.00 | 0.00 | 19.70 |
| | (B)*1 | | 0.24 | 0.24 | 0.24 | 0.24 | 0.24 | 0.24 | 0.24 | 0.24 | 0.24 |
| | (X)*2 | | 1.06 | 1.06 | 1.06 | 1.06 | 1.06 | 1.06 | 1.06 | 1.06 | 1.06 |
| Mass ratio | (A>/(B> | | 112.92 | 189.58 | 217.08 | 244.17 | 317.08 | 342.50 | 400.00 | 0.00 | 82.08 |
| | (A)/(X) | | 25.57 | 42.92 | 49.15 | 55.28 | 71.79 | 77.55 | 90.57 | 0.00 | 18.58 |
| | (A)/(D) | | 0.38 | 0.86 | 1.13 | 1.48 | 3.44 | 5.12 | 42.67 | 0.00 | 0.25 |
| | (B)/(X) | | 0.23 | 0.23 | 0.23 | 0.23 | 0.23 | 0.23 | 0.23 | 0.23 | 0.23 |
| Molar ratio | (X)/(B) | | 66.06 | 66.06 | 66.06 | 66.06 | 66.06 | 66.06 | 66.06 | 66.06 | 66.06 |
| Evaluation | Viscosity (mPa•s) | | 18.9 | 28.7 | 32.7 | 32.4 | 48.6 | 54.1 | 73.9 | 1.0 | 1.0 |
| | Appearance | | 3 | 3 | 3 | 3 | 3 | 3 | 3 | 3 | 3 |
| | Storage stability (20°C, 1 week) | | 3 | 3 | 3 | 3 | 3 | 3 | 3 | 3 | 3 |
| | Storage stability (20°C, 4 weeks) | | 3 | 3 | 3 | 3 | 3 | 3 | 3 | 3 | 3 |

Note *1: Amount in terms of a sulfate
*2: Amount in terms of a metal element
*3: Solid content: 100%

EP 3 239 284 A1

TABLE 2

| | | | Examples | | | | | | | Comparative Examples | |
|---|---|---|---|---|---|---|---|---|---|---|---|---|
| | | | 8 | 9 | 3 | 10 | 11 | 12 | 13 | 3 | 4 |
| Compounding amounts of respective components (%) | (A) | Ethylene glycol | 52.19 | 52.17 | 52.10 | 52.06 | 51.96 | 51.21 | 49.77 | 52.22 | 52.21 |
| | (B) | C22ESNa (EO = 4)*3 | 0.05 | 0.10 | 0.25 | 0.30 | 0.50 | 2.00 | 5.00 | 0.00 | 0.01 |
| | (C) | KOH*3 | 1.5 | 1.5 | 1.5 | 1.5 | 1.5 | 1.5 | 1.5 | 1.5 | 1.5 |
| | (D) | Water | 46.26 | 46.23 | 46.15 | 46.14 | 46.04 | 45.29 | 43.73 | 46.28 | 46.28 |
| | Total | | 100 | 100 | 100 | 100 | 100 | 100 | 100 | 100 | 100 |
| | (A) + (D) | | 98.45 | 98.40 | 98.25 | 98.20 | 98.00 | 96.50 | 93.50 | 98.50 | 98.49 |
| Contents of respective components (%) | (A) | | 52.19 | 52.17 | 52.10 | 52.06 | 51.96 | 51.21 | 49.77 | 52.22 | 52.21 |
| | (B)*1 | | 0.05 | 0.10 | 0.24 | 0.29 | 0.48 | 1.92 | 4.79 | 0.00 | 0.01 |
| | (X)*2 | | 1.05 | 1.05 | 1.06 | 1.06 | 1.07 | 1.13 | 1.25 | 1.04 | 1.05 |
| Mass ratio | (A)/(B) | | 1043.80 | 521.70 | 217.08 | 179.52 | 108.25 | 26.67 | 10.39 | - | 5221.00 |
| | (A)/(X) | | 49.70 | 49.69 | 49.15 | 49.11 | 48.56 | 45.32 | 39.82 | 50.21 | 49.72 |
| | (A)/(D) | | 1.13 | 1.13 | 1.13 | 1.13 | 1.13 | 1.13 | 1.14 | 1.13 | 1.13 |
| | (B)/(X) | | 0.05 | 0.10 | 0.23 | 0.27 | 0.45 | 1.70 | 3.83 | 0.00 | 0.01 |
| Molar ratio | (X)/(B) | | 312.85 | 156.96 | 66.06 | 54.86 | 33.58 | 9.23 | 4.36 | - | 1559.99 |
| Evaluation | Viscosity (mPa•s) | | 7.5 | 12.2 | 32.7 | 47.9 | 75.5 | 161.0 | 270.0 | 3.0 | 5.0 |
| | Appearance | | 3 | 3 | 3 | 3 | 3 | 3 | 3 | 0 | 0 |
| | Storage stability (20°C, 1 week) | | 3 | 3 | 3 | 3 | 3 | 3 | 3 | 0 | 0 |
| | Storage stability (20°C, 4 weeks) | | 3 | 3 | 3 | 3 | 3 | 3 | 3 | 0 | 0 |

Note *1: Amount in terms of a sulfate
*2: Amount in terms of a metal element
*3: Solid content: 100%

EP 3 239 284 A1

TABLE 3

| | | | Examples | | | | Comparative Examples | |
|---|---|---|---|---|---|---|---|---|
| | | | 14 | 15 | 3 | 16 | 5 | 6 |
| Compounding amounts of respective components (%) | (A) | Ethylene glycol | 52.8 | 52.3 | 52.1 | 51.8 | 52.9 | 50.8 |
| | (B) | C22ESNa (EO = 4)*3 | 0.25 | 0.25 | 0.25 | 0.25 | 0.25 | 0.25 |
| | (C) | KOH*3 | 0.2 | 1 | 1.5 | 2 | 0 | 4 |
| | (D) | Water | 46.75 | 46.45 | 46.15 | 45.95 | 46.85 | 44.95 |
| | Total | | 100 | 100 | 100 | 100 | 100 | 100 |
| | (A) + (D) | | 99.55 | 98.75 | 98.25 | 97.75 | 99.75 | 95.75 |
| Contents of respective components (%) | (A) | | 52.80 | 52.30 | 52.10 | 51.80 | 52.90 | 50.80 |
| | (B)*1 | | 0.24 | 0.24 | 0.24 | 0.24 | 0.24 | 0.24 |
| | (X)*2 | | 0.15 | 0.71 | 1.06 | 1.40 | 0.01 | 2.80 |
| Mass ratio | (A)/(B) | | 220.00 | 217.92 | 217.08 | 215.83 | 220.42 | 211.67 |
| | (A)/(X) | | 352.00 | 73.66 | 49.15 | 37.00 | 5290.00 | 18.14 |
| | (A)/(D) | | 1.13 | 1.13 | 1.13 | 1.13 | 1.13 | 1.13 |
| | (B)/(X) | | 1.60 | 0.34 | 0.23 | 0.17 | 24.00 | 0.09 |
| Molar ratio | (X)/(B) | | 9.77 | 44.41 | 66.06 | 87.71 | 1.10 | 174.32 |
| Evaluation | Viscosity (mPa·s) | | 16.1 | 31.9 | 32.7 | 30.17 | 4.0 | 8.0 |
| | Appearance | | 3 | 3 | 3 | 3 | 3 | 1 |
| | Storage stability (20°C, 1 week) | | 3 | 3 | 3 | 3 | 3 | 1 |
| | Storage stability (20°C, 4 weeks) | | 3 | 3 | 3 | 3 | 3 | 1 |
| Note *1: Amount in terms of a sulfate *2: Amount in terms of a metal element *3: Solid content: 100% | | | | | | | | |

TABLE 4

| | | | Example 17 | Example 18 |
|---|---|---|---|---|
| Compounding amounts of respective components (%) | (A) | Ethylene glycol | 52.10 | 52.10 |
| | (B) | C18ESNa (EO = 4)*3 | 0.25 | - |
| | | C18ESNa (EO = 1.5)*3 | - | 0.25 |
| | (C) | KOH*3 | 1.50 | 1.50 |
| | (D) | Water | 46.15 | 46.15 |
| | Total | | 100 | 100 |
| | (A) + (D) | | 98.25 | 98.25 |

(continued)

| | | Example 17 | Example 18 |
|---|---|---|---|
| Contents of respective components (%) | (A) | 52.10 | 52.10 |
| | (B)*1 | 0.24 | 0.24 |
| | (X)*2 | 1.06 | 1.06 |
| Mass ratio | (A)/(B) | 217.08 | 217.08 |
| | (A)/(X) | 49.15 | 49.15 |
| | (A)/(D) | 1.13 | 1.13 |
| | (B)/(X) | 0.23 | 0.23 |
| Molar ratio | (X)/(B) | 60.17 | 48.23 |
| Evaluation | Viscosity (mPa•s) | 17.9 | 8.9 |
| | Appearance | 3 | 3 |
| | Storage stability (20°C, 1 week) | 2 | 3 |
| | Storage stability (20°C, 4 weeks) | 2 | 3 |
| Note *1: Amount in terms of a sulfate *2: Amount in terms of a metal element *3: Solid content: 100% | | | |

TABLE 5-1

| | | | Example 3 | Comparative Examples | | Example 17 | Comparative Examples | |
|---|---|---|---|---|---|---|---|---|
| | | | | 7 | 8 | | 9 | 10 |
| Compounding amounts of respective components (%) | (A) | Ethylene glycol | 52.10 | 52.10 | - | 52.10 | 52.20 | - |
| | (B) | C12ESNa (EO = 3)*3 | - | - | - | - | - | - |
| | | C16ESNa (EO = 4)*3 | - | - | - | - | - | - |
| | | C18ESNa (EO = 4)*3 | - | - | - | 0.25 | - | 0.25 |
| | | C22ESNa (EO = 4)*3 | 0.25 | - | 0.25 | - | - | - |
| | (C) | KOH*3 | 1.50 | 1.50 | - | 1.50 | 1.50 | - |
| | (D) | Water | 46.15 | 46.40 | 99.75 | 46.15 | 46.30 | 99.75 |
| | Total | | 100 | 100 | 100 | 100 | 100 | 100 |
| | (A) + (D) | | 98.25 | 98.50 | 99.75 | 98.25 | 98.50 | 99.75 |
| Contents of respective components (%) | (A) | | 52.10 | 52.10 | 0.00 | 52.10 | 52.20 | 0.00 |
| | (B)*1 | | 0.24 | 0.00 | 0.24 | 0.24 | 0.00 | 0.24 |
| | (X)*2 | | 1.06 | 1.04 | 0.01 | 1.06 | 1.04 | 0.01 |

(continued)

| | | Example 3 | Comparative Examples | | Example 17 | Comparative Examples | |
|---|---|---|---|---|---|---|---|
| | | | 7 | 8 | | 9 | 10 |
| Mass ratio | (A)/(B) | 217.08 | - | 0.00 | 217.08 | - | 0.00 |
| | (A)/(X) | 49.15 | 50.10 | 0.00 | 49.15 | 50.20 | 0.00 |
| | (A)/(D) | 1.13 | 1.13 | 0.00 | 1.13 | 1.13 | 0.00 |
| | (B)/(X) | 0.23 | 0.00 | 24.00 | 0.23 | 0.00 | 24.00 |
| Molar ratio | (X)/(B) | 66.06 | - | 1.10 | 60.17 | - | 1.10 |
| Evaluation | Viscosity (mPa•s) | 32.7 | 3.0 | 1.0 | 17.9 | 3.0 | 1.0 |
| | Appearance | 3 | 0 | 3 | 3 | 0 | 3 |
| | Storage stability (20°C, 1 week) | 3 | 0 | 3 | 2 | 0 | 3 |
| | Storage stability (20°C, 4 weeks) | 3 | 0 | 3 | 2 | 0 | 3 |

Note *1: Amount in terms of a sulfate
*2: Amount in terms of a metal element
*3: Solid content: 100%

TABLE 5-2

| | | | Example 19 | Comparative Examples | | | | |
|---|---|---|---|---|---|---|---|---|
| | | | | 11 | 12 | 13 | 14 | 15 |
| Compounding amounts of respective components (%) | (A) | Ethylene glycol | 52.10 | 52.20 | - | 52.10 | 52.20 | 49.57 |
| | (B) | C12ESNa (EO = 3)*3 | - | - | - | 0.25 | - | 5.00 |
| | | C16ESNa (EO = 4)*3 | 0.25 | - | 0.25 | - | - | - |
| | | C18ESNa (EO = 4)*3 | - | - | - | - | - | - |
| | | C22ESNa (EO = 4)*3 | - | - | - | - | - | - |
| | (C) | KOH*3 | 1.50 | 1.50 | - | 1.50 | 1.50 | 1.50 |
| | (D) | Water | 46.15 | 46.30 | 99.75 | 46.15 | 46.30 | 43.93 |
| | Total | | 100 | 100 | 100 | 100 | 100 | 100 |
| | (A) + (D) | | 98.25 | 98.50 | 99.75 | 98.25 | 98.50 | 93.50 |
| Contents of respective components (%) | (A) | | 52.10 | 52.20 | 0.00 | 52.10 | 52.20 | 49.75 |
| | (B)*1 | | 0.24 | 0.00 | 0.24 | 0.24 | 0.00 | 4.70 |
| | (X)*2 | | 1.06 | 1.04 | 0.01 | 1.06 | 1.04 | 1.29 |

(continued)

| | | Example 19 | Comparative Examples | | | | |
|---|---|---|---|---|---|---|---|
| | | | 11 | 12 | 13 | 14 | 15 |
| Mass ratio | (A)/(B) | 217.08 | - | 0.00 | 217.08 | - | 10.54 |
| | (A)/(X) | 49.15 | 50.20 | 0.00 | 49.15 | 50.20 | 38.43 |
| | (A)/(D) | 1.13 | 1.13 | 0.00 | 1.13 | 1.13 | 1.13 |
| | (B)/(X) | 0.23 | 0.00 | 24.00 | 0.23 | - | 3.64 |
| Molar ratio | (X)/(B) | 56.66 | - | 1.10 | 45.50 | - | 3.37 |
| Evaluation | Viscosity (mPa•s) | 17.1 | 3.0 | 1.0 | 4.0 | 3.0 | 4.0 |
| | Appearance | 3 | 0 | 3 | 0 | 0 | 0 |
| | Storage stability (20°C, 1 week) | 2 | 0 | 3 | 0 | 0 | 0 |
| | Storage stability (20°C, 4 weeks) | 2 | 0 | 3 | 0 | 0 | 0 |

TABLE 6-1

| | | | Examples | | | | | Comparative Examples | | |
|---|---|---|---|---|---|---|---|---|---|---|
| | | | 3 | 20 | 21 | 22 | 23 | 16 | 17 | 18 |
| Compounding amounts of respective components (%) | (A) | Ethylene glycol | 52.10 | - | - | 52.10 | 52.10 | - | 52.10 | 52.10 |
| | | 1,2-Propanediol | - | 52.10 | - | - | - | - | - | - |
| | | Glycerin | - | - | 52.10 | - | - | - | - | - |
| | (A')[4] | Ethanol | - | - | - | - | - | 52.10 | - | - |
| | (B) | C22ESNa (EO = 4)[3] | 0.25 | 0.25 | 0.25 | 0.25 | 0.25 | 0.25 | 0.25 | 0.25 |
| | (C) | KOH[3] | 1.50 | 1.50 | 1.50 | - | - | 1.50 | - | - |
| | | NaOH[3] | - | - | - | 1.50 | - | - | - | - |
| | | KCL[3] | - | - | - | - | 2.30 | - | - | - |
| | (C')[5] | Ca(OH)$_2$[3] | - | - | - | - | - | - | 1.98 | - |
| | | Triethanol amine | - | - | - | - | - | - | - | 3.99 |
| | (D) | Water | 46.15 | 46.15 | 46.15 | 46.15 | 45.35 | 46.15 | 45.67 | 43.66 |
| | Total | | 100 | 100 | 100 | 100 | 100 | 100 | 100 | 100 |
| | (A) + (D) | | 98.25 | 98.25 | 98.25 | 98.25 | 97.45 | 98.25 | 97.77 | 95.76 |
| Contents of respective components (%) | (A) | | 52.10 | 52.10 | 52.10 | 52.10 | 52.10 | 52.10 | 52.10 | 52.10 |
| | (B)[1] | | 0.24 | 0.24 | 0.24 | 0.24 | 0.24 | 0.24 | 0.24 | 0.24 |
| | (X)[2] | | 1.06 | 1.06 | 1.06 | 0.87 | 1.22 | 1.06 | 1.08 | 4.00 |

Note *1: Amount in terms of a sulfate
*2: Amount in terms of a metal element
*3: Solid content: 100%
*4: Solvent other than component (A)
*5: Alkali agent other than component (C)

EP 3 239 284 A1

22

TABLE 6-2

| | | Examples | | | | | Comparative Examples | | |
|---|---|---|---|---|---|---|---|---|---|
| | | 3 | 20 | 21 | 22 | 23 | 16 | 17 | 18 |
| Mass ratio | (A)/(B) | 217.08 | 217.08 | 217.08 | 217.08 | 217.08 | 217.08 | 217.08 | 217.08 |
| | (A)/(X) | 49.15 | 49.15 | 49.15 | 59.88 | 42.70 | 49.15 | 48.24 | 13.02 |
| | (A)/(D) | 1.13 | 1.13 | 1.13 | 1.13 | 1.13 | 1.13 | 1.14 | 1.19 |
| | (B)/(X) | 0.23 | 0.23 | 0.23 | 0.27 | 0.20 | 0.23 | 0.22 | 0.06 |
| Molar ratio | (X)/(B) | 66.06 | 66.06 | 66.06 | 92.04 | 76.64 | 66.06 | 63.35 | 63.40 |
| Evaluation | Viscosity (mPa•s) | 32.7 | 37.0 | 18.7 | 18.7 | 32.5 | 3.0 | 3.0 | 5.0 |
| | Appearance | 3 | 3 | 3 | 3 | 3 | 1 | 1 | 0 |
| | Storage stability (20°C, 1 week) | 3 | 3 | 3 | 3 | 3 | 1 | 1 | 0 |
| | Storage stability (20°C, 4 weeks) | 3 | 3 | 3 | 3 | 3 | 1 | 1 | 0 |

As apparently recognized from Tables 1 to 6, the surfactant composition of the present invention exhibits a high viscosity even when the concentration of a surfactant therein is low, and is also excellent in appearance and storage stability.

In addition, with respect to the surfactant composition obtained in Example 13, the resulting aggregate had such a structure as shown in a polarization microphotograph of FIG. 1.

INDUSTRIAL APPLICABILITY

The surfactant composition of the present invention exhibits a high viscosity, and is also excellent in appearance and storage stability. The surfactant composition of the present invention may be suitably used in the applications including cosmetics, detergents, coating materials, inks, lubricants, adhesives, agricultural compounding agents, drilling fluids for petroleum, etc.

**Claims**

1. A surfactant composition comprising:

   a component (A): a polyol having not less than 2 and not more than 3 carbon atoms;
   a component (B): a polyoxyalkylene alkyl or alkenyl ether sulfate or a salt thereof represented by the following general formula (I):

   $$R^1O\text{-}(R^2O)_mSO_3M \qquad (I)$$

   wherein $R^1$ is an alkyl or alkenyl group having not less than 16 and not more than 24 carbon atoms; $R^2O$ is an oxyalkylene group; m represents an average molar number of addition of an alkyleneoxide, and is a number of not less than 0.5 and not more than 10; and M is a hydrogen atom or a cation;
   a component (C): at least one compound selected from the group consisting of an alkali metal salt other than the component (B) and an alkali metal hydroxide; and
   a component (D): water,

   in which a content of the component (A) in the composition is not less than 25% by mass and not more than 98% by mass,

a content of the component (B) in the composition is not less than 0.05% by mass and not more than 10% by mass in terms of the sulfate,

a total content of alkali metals (X) derived from the polyoxyalkylene alkyl or alkenyl ether sulfate salt as the component (B) and derived from the at least one compound selected from the group consisting of the alkali metal salt and the alkali metal hydroxide as the component (C), in the composition is not more than 2.5% by mass in terms of the metal elements, and

a molar ratio of the alkali metals (X) to the component (B) [(X)/(B)] is not less than 1.5.

2. The surfactant composition according to claim 1, wherein a total content of the components (A) and (D) in the composition is not less than 85% by mass and not more than 99.6% by mass.

3. The surfactant composition according to claim 1 or 2, wherein a content of the component (D) in the composition is not less than 2% by mass and not more than 72% by mass.

4. The surfactant composition according to any one of claims 1 to 3, wherein $R^2O$ is an oxyethylene group.

5. The surfactant composition according to any one of claims 1 to 4, wherein a mass ratio of the component (A) to the component (B) [(A)/(B)] is not less than 2.5 and not more than 1,900.

6. The surfactant composition according to any one of claims 1 to 5, wherein a mass ratio of the component (A) to the alkali metals (X) [(A)/(X)] is not less than 20 and not more than 1,000.

7. The surfactant composition according to any one of claims 1 to 6, wherein a mass ratio of the component (A) to the component (D) [(A)/(D)] is not less than 0.35 and not more than 45.

8. The surfactant composition according to any one of claims 1 to 7, wherein a molar ratio of the alkali metals (X) to the component (B) [(X)/(B)] is not less than 1.5 and not more than 3,000.

9. The surfactant composition according to any one of claims 1 to 8, wherein the total content of the alkali metals (X) in the composition is not less than 0.05% by mass and not more than 2.5% by mass in terms of the metal elements.

10. The surfactant composition according to any one of claims 1 to 9, wherein a viscosity of the composition as measured at 25°C is not less than 6 mPa•s.

11. The surfactant composition according to any one of claims 1 to 10, wherein the composition is produced by compounding the components (A), (B), (C) and (D).

12. A process for producing the surfactant composition according to any one of claims 1 to 11, comprising the step of compounding the components (A), (B), (C) and (D).

Fig. 1

Lens Z100 : X1000                    50.00 μm

**INTERNATIONAL SEARCH REPORT**

| International application No. |
| --- |
| PCT/JP2015/084311 |

A. CLASSIFICATION OF SUBJECT MATTER

*C11D3/20*(2006.01)i, *A61K8/19*(2006.01)i, *A61K8/34*(2006.01)i, *A61K8/46* (2006.01)i, *C11D1/29*(2006.01)i, *C11D3/04*(2006.01)i

According to International Patent Classification (IPC) or to both national classification and IPC

B. FIELDS SEARCHED

Minimum documentation searched (classification system followed by classification symbols)
C11D3/20, A61K8/19, A61K8/34, A61K8/46, C11D1/29, C11D3/04

Documentation searched other than minimum documentation to the extent that such documents are included in the fields searched
| | | | |
| --- | --- | --- | --- |
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)
CAplus/REGISTRY(STN), JSTPlus/JMEDPlus/JST7580/JSTChina(JDreamIII)

C. DOCUMENTS CONSIDERED TO BE RELEVANT

| Category* | Citation of document, with indication, where appropriate, of the relevant passages | Relevant to claim No. |
| --- | --- | --- |
| A | JP 2009-185252 A (Kao Corp.), 20 August 2009 (20.08.2009), claims; paragraphs [0076] to [0102] (Family: none) | 1-12 |
| A | JP 8-183729 A (Kao Corp.), 16 July 1996 (16.07.1996), claims; paragraph [0057] (Family: none) | 1-12 |
| A | JP 2010-13579 A (Kao Corp.), 21 January 2010 (21.01.2010), claims; paragraphs [0064] to [0070] (Family: none) | 1-12 |

| ☒ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 23 February 2016 (23.02.16) | 08 March 2016 (08.03.16) |

| Name and mailing address of the ISA/ | Authorized officer |
| --- | --- |
| Japan Patent Office 3-4-3,Kasumigaseki,Chiyoda-ku, Tokyo 100-8915,Japan | |
| | Telephone No. |

Form PCT/ISA/210 (second sheet) (January 2015)

**INTERNATIONAL SEARCH REPORT**

International application No.

PCT/JP2015/084311

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2013-60423 A  (Lion Corp.), 04 April 2013 (04.04.2013), claims; paragraphs [0048], [0059] to [0060] (Family: none) | 1-12 |
| A | JP 2002-129189 A  (Teepol Ltd.), 09 May 2002 (09.05.2002), claims; paragraph [0063] (Family: none) | 1-12 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009120664 A
- JP 61081496 A